(19) Europäisches Patentamt
European Patent Office
Office européen des brevets

(11) Veröffentlichungsnummer: **0 285 548 B1**

(12) # EUROPÄISCHE PATENTSCHRIFT

(45) Veröffentlichungstag der Patentschrift: **05.09.90**

(51) Int. Cl.⁵: **C07J 1/00**, C07J 13/00

(21) Anmeldenummer: **88730075.4**

(22) Anmeldetag: **25.03.88**

(54) **Verfahren zur Herstellung von 17alpha-Ethinyl-17beta-hydroxy-18-methyl-4,15-estradien-3-on und die neuen Zwischenprodukte für dieses Verfahren.**

(30) Priorität: **31.03.87 DE 3710728**

(43) Veröffentlichungstag der Anmeldung: **05.10.88 Patentblatt 88/40**

(45) Bekanntmachung des Hinweises auf die Patenterteilung: **05.09.90 Patentblatt 90/36**

(84) Benannte Vertragsstaaten: **AT BE CH DE ES FR GB GR IT LI LU NL SE**

(56) Entgegenhaltungen:
**EP-A- 0 201 452**
**DE-A- 2 546 062**
**FR-A- 1 476 573**

(73) Patentinhaber: **SCHERING AKTIENGESELLSCHAFT Berlin und Bergkamen, Müllerstrasse 170/178 Postfach 65 03 11, D-1000 Berlin 65(DE)**

(72) Erfinder: **Bohlmann, Rolf, Dr., Melanchtonstrasse 23, D-1000 Berlin 21(DE)**
Erfinder: **Laurent, Henry, Dr., Glambecker Weg 21, D-1000 Berlin 28(DE)**
Erfinder: **Hofmeister, Helmut, Dr., Weislingenstrasse 4, D-1000 Berlin 28(DE)**
Erfinder: **Wiechert, Rudolf, Prof. Dr., Petzower Strasse 8a, D-1000 Berlin 39(DE)**

**EP 0 285 548 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von 17α-Ethinyl-17β-hydroxy-18-methyl-4,15-estradien-3-on (Gestoden) und die neuen Zwischenverbindungen für dieses Verfahren. Gestoden ist ein stark wirksames Gestagen, das zum Beispiel als gestagener Bestandteil in Präparaten zur Kontrazeption verwendet werden kann (DE-PS 25 46 062).

Es sind bereits mehrere Verfahren zur Herstellung von Gestoden bekannt. Die Einführung der $\Delta^{15}$-Doppelbindung ist jedoch als noch nicht befriedigend gelöstes Problem anzusehen. Chemische Dehydrierungsmethoden verlaufen mit niedrigen Ausbeuten, da die 18-Methylgruppe die Reaktion hemmt (Deutsche Offenlegungsschrift 24 39 082). Dehydrierung unter Zuhilfenahme der Mikrobiologie zeichnen sich durch hohe Synthesekosten aus (Deutsche Offenlegungsschrift 24 56 068).

In Tetrahedron 42 (1986) 2971-2977 wird ein Verfahren zur Dehydrierung von Ketonen durch Palladium-katalysierte Umsetzungen von Enolacetaten mit Allylcarbonaten beschrieben.

Es wurde nun gefunden, daß sich die Palladium-katalysierte Dehydrierung gut auf die Einführung der $\Delta^{15}$-Doppelbindung beim Gestoden anwenden läßt. Das Verfahren hat den Vorteil, daß die Dehydrierung unter relativ milden Bedingungen und in höheren Ausbeuten als mit den bekannten Verfahren verläuft.

Die Erfindung betrifft somit ein neues Verfahren zur Herstellung von Gestoden, dadurch gekennzeichnet, daß man Verbindungen der allgemeinen Formel II

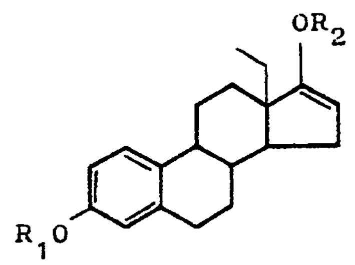

II,

worin
$R_1$ einen Alkylrest mit 1–3 Kohlenstoffatomen und
$R_2$ eine Acyl- oder eine Trialkylsilylgruppe mit bis zu 10 Kohlenstoffatomen
bedeuten,
unter Palladium-Katalyse in Verbindungen der allgemeinen Formel III

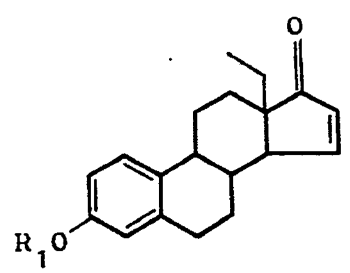

III,

wobei
$R_1$ einen Alkylrest mit 1–3 Kohlenstoffatomen bedeutet,
umsetzt und in an sich bekannter Weise die 17-Ketogruppe reduziert, den aromatischen A-Ring nach Birch mit flüssigem Ammoniak zum entsprechenden 3-Alkoxy-2,5(10),15-estratrien-17β-ol (Verbindung der allgemeinen Formel V des nachstehenden Reaktionsschemas) umsetzt, die 17-Hydroxygruppe zur 17-Ketogruppe rückoxidiert, die 17-Ketogruppe ethinyliert und den 3-Enolether spaltet.

In Formel II und III steht $R_1$ für einen Alkylrest mit 1 bis 3 Kohlenstoffatomen, wobei der Methyl- und Ethylrest bevorzugt sind. Als Acylreste $R_2$ in Formel II kommen die Reste organischer Carbonsäuren mit bis Acetyl-, Trifluoracetyl-, Trimethylacetyl-, Propionyl-, Butyryl-, Heptanoyl- und Benzoylrest, wobei der Acetylrest bevorzugt ist. Als Trialkylsilylgruppe wird insbesondere die Trimethylsilylgruppe angewendet.

17-Acetoxy-3-methoxy-18-methyl-1,3,5(10),16-estratetraen, eine Verbindung der allgemeinen Formel II, ist bereits in der FR-A 1 476 573 beschrieben.

Die Palladium-katalysierte Umsetzung der entsprechenden Enolacetate gelingt mit Trialkylsilyl- oder Trialkylzinnalkoxid und Allylcarbonat in guten Ausbeuten. Als Lösungsmittel kommen Nitrile, insbesondere Benzonitril und Acetonitril infrage.

Geeignete Palladiumkatalysatoren sind Palladiummetall, Palladiumverbindungen und -salze, wie zum Beispiel Palladiumacetat, und Palladiumkomplexe, wie zum Beispiel Bis(di-1,2-diphenylphosphinethan)palladium(0) oder Tetrakis(triphenylphosphin)palladium(0).

Als Trialkylsilylgruppe kommt vorzugsweise eine Trimethylsilyl-, eine Tribenzylsilyl- oder eine Dimethyl-tert-butylsilylgruppe und als Trialkylzinngruppe kommt vorzugsweise eine Tripropyl-, eine Tributyl- oder Tripentylzinngruppe infrage.

Das Alkoxid im Trialkylsilyl- oder Trialkylzinnalkoxid ist vorzugsweise ein Methoxid, Ethoxid oder Propoxid.

Nach einer bevorzugten Ausführungsform wird das Enolderivat in Acetonitril mit katalytischen Mengen Palladiumacetat (2–20 g/Mol Enolderivat) sowie katalytischen Mengen Tributylzinnmethoxid (4–60 g/Mol Enolderivat) in Gegenwart von Allylmethylcarbonat im Überschuß (20–200 g/Mol Enolderivat) unter Erhitzen am Rückfluß (60–140°C) behandelt. Nach einer Reaktionszeit von 1–3 Stunden kann das dehydrierte Produkt abgetrennt und chromatographisch gereinigt werden.

Die Weiterverarbeitung der Verbindungen der allgemeinen Formel III zu Gestoden der Formal I sei anhand des folgenden Formelschemas näher erläutert:

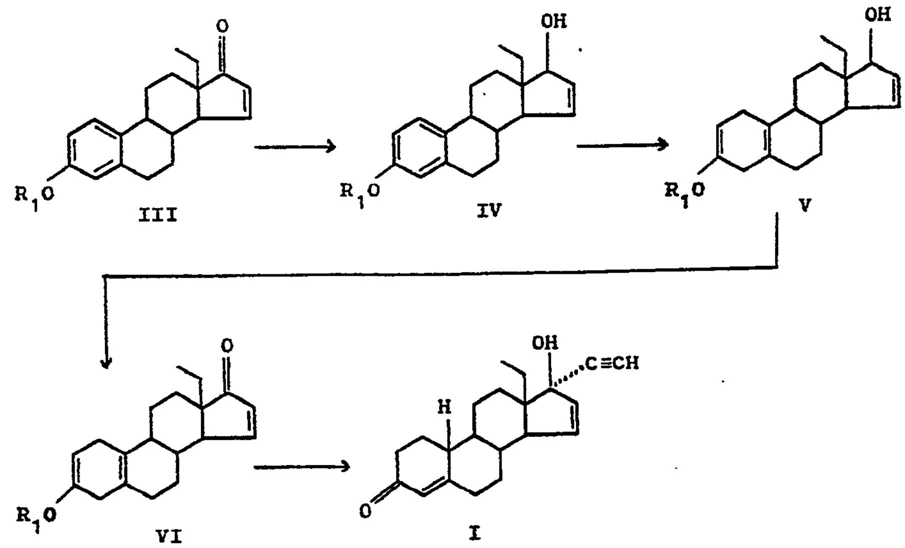

III IV V VI I

Das 17-Keton wird reduziert, zum Beispiel mit Natriumborhydrid in Anwesenheit von Cer(III)-Ionen; nach Birch-Reduktion des aromatischen A-Ringes, Oxidation des 17β-Alkohols, zum Beispiel mit Braunstein, Ethinylierung des 17-Ketons und anschließende saure Enoletherspaltung wird Gestoden der Formel I erhalten.

Die Reduktion mit Natriumborhydrid in Anwesenheit von Cer(III)-Ionen erfolgt in an sich bekannter Weise, zum Beispiel analog dem in J. Am. Chem. Soc. 100 (1978) 2226 angegebenen Verfahren. Nach bekannten Verfahren verlaufen auch die Birch-Reduktion [J. Am. Chem. Soc. 75 (1953) 5366], die Oxydation mit Braunstein [J. Am. Chem. Soc. 77 (1955) 4145] sowie die Ethinylierung und Enoletherspaltung [Org. Synth. Coll. 3 (1955) 416].

**Beispiel 1**

<u>17-Acetoxy-3-methoxy-18-methyl-1,3,5(10),16-estratetraen</u>

30 g 3-Methoxy-18-methyl-1,3,5(10)-estratrien-17-on [J. Org. Chem. <u>40</u> (1975) 681] werden in 200 ml Isopropenylacetat mit 1,5 g p-Toluolsulfonsäure 22 Stunden bei 120 °C Badtemperatur gerührt. Dann wird mit Essigester verdünnt, mit Natriumcarbonat und Kochsalzlösung gewaschen, über Natriumsulfat getrocknet und eingeengt. Nach Chromatographie erhält man 27,6 g 17-Acetoxy-3-methoxy-18-methyl-1,3,5(10),16-estratetraen vom Schmelzpunt 105 - 106 °C.

**Beispiel 2**

<u>3-Methoxy-18-methyl-1,3,5(10),15-estratetraen-17-on</u>

3,4 g 17-Acetoxy-3-methoxy-18-methyl-1,3,5(10),16-estratetraen werden in 50 ml Acetonitril mit 2,3 ml Allylmethylcarbonat, 220 mg Palladiumacetat und 580 µl Tributylzinnmethoxid 1,5 Stunden am Rückfluß erhitzt. Dann wird mit Wasser verdünnt, mit Dichlormethan extrahiert und im Vakuum eingeengt. Nach chromatographischer Reinigung erhält man 2,1 g 3-Methoxy-18-methyl-1,3,5(10),15-estratetraen-17-on vom Schmelzpunkt 158 - 160 °C.

**Beispiel 3**

3-Methoxy-18-methyl-1,3,5(10),15-estratetraen-17β-ol

4,7 g 3-Methoxy-18-methyl-1,3,5(10),15-estratetraen-17-on in 30 ml Tetrahydrofuran und 45 ml Methanol werden mit 6,8 g Cer(III)-chlorid Heptahydrat versetzt. Bei 0 °C gibt man portionsweise 1,0 g Natriumborhydrid zu. Nach 1 Stunde wird das Reaktionsgemisch in Eis/Wasser gegeben. Das ausgefallene Produkt wird abgesaugt, in Essigester gelöst, mit Wasser gewaschen und getrocknet. Es werden 4,5 g 3-Methoxy-18-methyl-1,3,5(10),15-estratetraen-17β-ol als Schaum erhalten.

**Beispiel 4**

3-Methoxy-18-methyl-2,5(10),15-estratrien-17β-ol

5,0 g 3-Methoxy-18-methyl-1,3,5(10),15-estratetraen-17β-ol in 250 ml Tetrahydrofuran werden bei -78 °C zu 250 ml flüssigem Ammoniak gegeben. Man versetzt mit 30 ml Ethanol und gibt 1,5 g Lithium in kleinen Portionen hinzu. Nach vollständiger Umsetzung läßt man den Ammoniak verdampfen, tropft unter Kühlung vorsichtig Wasser zu, verdünnt mit Essigester, wäscht die organische Phase mit Wasser neutral und trocknet. Man erhält 4,7 g 3-Methoxy-18-methyl-2,5(10),15-estratrien-17β-ol als Schaum.

**Beispiel 5**

3-Methoxy-18-methyl-2,5(10),15-estratrien-17-on

4,7 g 3-Methoxy-18-methyl-2,5(10),15-estratrien-17β-ol in 250 ml Chloroform und 35 ml t-Butanol werden mit 10 g Mangandioxid 18 Stunden am Rückfluß erhitzt. Dann wird über Celite abgesaugt, mit Chloroform nachgewaschen und unter vermindertem Druck eingeengt. Nach der chromatographischen Reinigung an Kieselgel mit Hexan/Essigester erhält man 4,1 g reines 3-Methoxy-18-methyl-2,5(10),15-estratrien-17-on vom Schmelzpunkt 89 - 91 °C.

**Beispiel 6**

17α-Ethinyl-17β-hydroxy-18-methyl-4,15-estradien-3-on (Gestoden)

In eine Lösung von 40 ml n-Butyllithium (15 %ig in Hexan) in 100 ml Tetrahydrofuran leitet man bei 0 °C 30 Minuten Acetylen ein und tropft dann 4,0 g 3-Methoxy-2,5(10),15-estratrien-17-on in 40 ml Tetrahydrofuran zu. Nach 45 Minuten versetzt man das Reaktionsgemisch mit 16 ml halbkonzentrierter Salzsäure und rührt 45 Minuten bei Raumtemperatur. Es wird mit Essigester verdünnt, mit Wasser neutral gewaschen und getrocknet. Es werden nach dem Umkristallisieren aus Essigester 2,4 g 17α-Ethinyl-17β-hydroxy-18-methyl-4,15-estradien-3-on vom Schmelzpunkt 196 °C erhalten.

**Patentansprüche**

1.) Verfahren zur Herstellung von 17α-Ethinyl-17β-hydroxy-18-methyl-4,15-estradien-3-on der Formel I

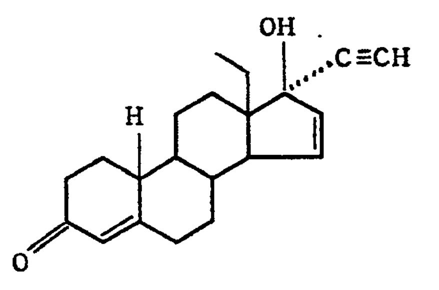

I,

dadurch gekennzeichnet, daß man einen 17-Enolester der allgemeinen Formel II

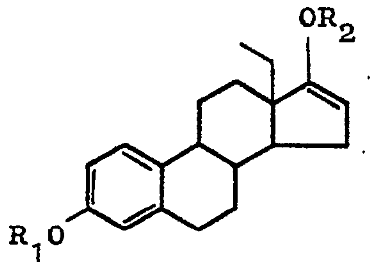

II.

worin
$R_1$ einen Alkylrest mit 1 - 3 Kohlenstoffatomen und
$R_2$ eine Acyl- oder eine Trialkylsilylgruppe mit bis zu 10 Kohlenstoffatomen bedeuten,
unter Palladium-Katalyse in Verbindungen der allgemeinen Formel III

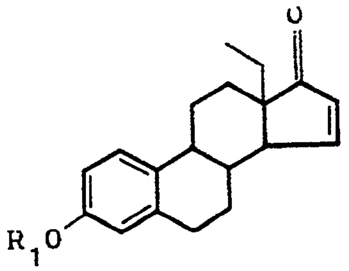

III.

wobei
$R_1$ einen Alkylrest mit 1–3 Kohlenstoffatomen bedeutet,
umsetzt und in an sich bekannter Weise die 17-Ketogruppe reduziert, den aromatischen A-Ring nach Birch mit flüssigem Ammoniak zum entsprechenden 3-Alkoxy-2,5(10),15-estratrien-17β-ol umsetzt, die17-Hydroxygruppe zur 17-Ketogruppe rückoxidiert, die 17-Ketogruppe ethinyliert und den 3-Enolether spaltet.

2. Verbindungen der allgemeinen Formel III

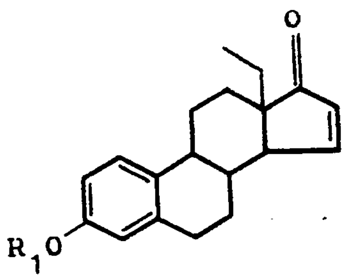

III.

worin
$R_1$ einen Alkylrest mit 1–3 Kohlenstoffatomen bedeutet.

3. 3-Methoxy-18-methyl-1,3,5(10),15-estratetraen-17-on nach Anspruch 2.
4. 3-Methoxy-18-methyl-1,3,5(10),15-estratetraen-17β-ol.
5. 3-Methoxy-18-methyl-2,5(10),15-estratrien-17β-ol.
6. 3-Methoxy-18-methyl-2,5(10),15-estratrien-17-on.

## Claims

1. Process for the manufacture of 17α-ethynyl-17β-hydroxy-18-methyl-4,15-estradien-3-one of the formula I

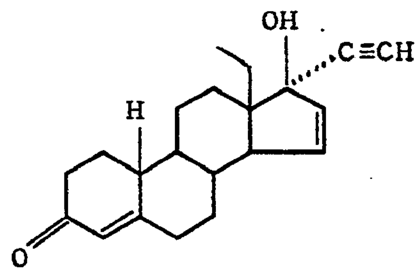

I,

characterised in that a 17-enol ester of the general formula II

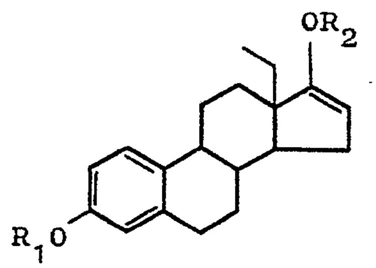

II,

wherein
$R_1$ represents an alkyl radical having 1–3 carbon atoms and
$R_2$ represents an acyl or a trialkylsilyl group having up to 10 carbon atoms,
is reacted with palladium catalysis to form compounds of the general formula III

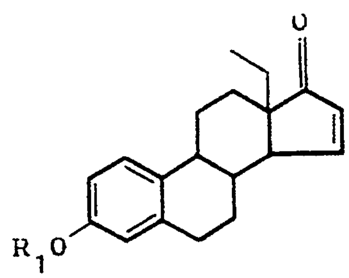

III,

wherein
$R_1$ represents an alkyl radical having 1–3 carbon atoms,
and, in a manner known per se, the 17-keto group is reduced, the aromatic A-ring is reacted by the Birch reaction with liquid ammonia to form the corresponding 3-alkoxy-2,5(10),15-estratrien-17β-ol, the 17-hydroxy group is reoxidised to the 17-keto group, the 17-keto group is ethynylated and the 3-enol ether is cleaved.

2. Compounds of the general formula III

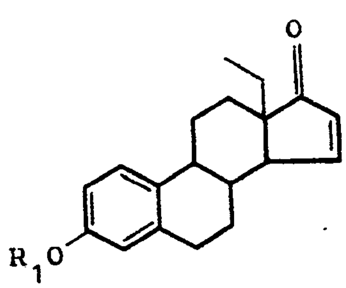

III,

wherein
$R_1$ represents an alkyl radical having 1–3 carbon atoms.

3. 3-Methoxy-18-methyl-1,3,5(10),15-estratetraen-17-one according to claim 2.
4. 3-Methoxy-18-methyl-1,3,5(10),15-estratetraen-17β-ol.
5. 3-Methoxy-18-methyl-2,5(10),15-estratrien-17β-ol.
6. 3-Methoxy-18-methyl-2,5(10),15-estratrien-17-one.

## Revendications

1. Procédé de préparation de 17α-éthynyl-17β-hydroxy-18-méthyl-4,15-oestradién-3-one répondant à la formule I

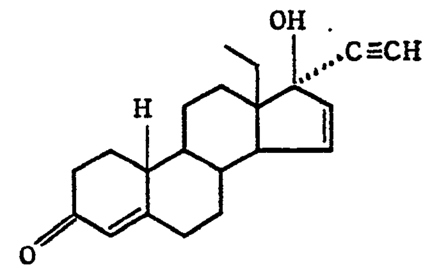

I,

caractérisé en ce qu'on convertit un 17-énolester de la formule générale II

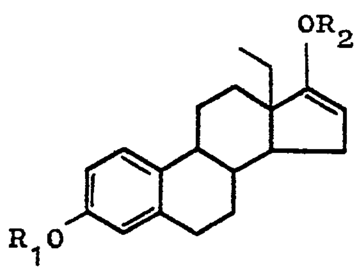

II,

dans laquelle
$R_1$ représente un radical alkyle comportant 1 à 3 atomes de carbone et $R_2$ représente un groupe acyle ou trialkylsilyle comportant jusqu'à 10 atomes de carbone, en composés de la formule générale III

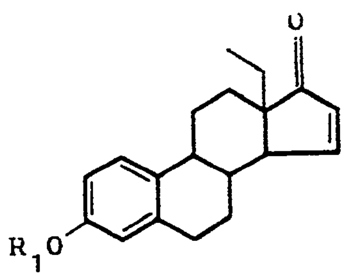

III,

dans laquelle
$R_1$ représente un radical alkyle comportant 1 à 3 atomes de carbone, avec une catalyse au palladium, et, d'une manière connue en soi, on réduit le groupe 17-cétonique, on convertit le noyau A aromatique selon Birch avec de l'ammoniac liquide pour former le 3-alcoxy-2,5(10),15-oestratrién-17β-ol correspondant, on réoxyde le groupe 17-hydroxy en le groupe 17-cétonique, on éthynyle le groupe 17-cétonique, et on sépare le 3-énoléther.

2. Composés répondant à la formule générale III

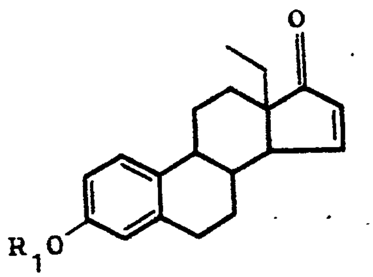

III,

dans laquelle
$R_1$ représente un radical alkyle comportant 1 à 3 atomes de carbone.

3. 3-Méthoxy-18-méthyl-1,3,5(10),15-oestratétraén-17-one selon la revendication 2.
4. 3-Méthoxy-18-méthyl-1,3,5(10),15-oestratétraén-17β-ol.
5. 3-Méthoxy-18-méthyl-2,5(10),15-oestratrién-17β-ol.
6. 3-Méthoxy-18-méthyl-2,5(10),15-oestratrién-17-one.